# EUROPEAN PATENT APPLICATION

(11) **EP 2 161 047 A1**
(43) Date of publication of application: **10.03.2010**
(21) Application number: 08787626.4
(22) Date of filing: 24.06.2008
(51) Int. Cl.: A61M 16/04, A61F 13/02, A61F 15/00

(54) **STOMA PROTECTOR**

(30) Priority: 26.06.2007 ES 200701795
(71) Applicant: Pino Aragones, Antonio Del, 28760 Tres Cantos Madrid (ES)
(72) Inventor: Pino Aragones, Antonio Del, 28760 Tres Cantos Madrid (ES)
(74) Representative: Pereira Toña, Maria Irache
(86) International application number: PCT/ES2008/000445
(87) International publication number: WO 2009/000952

(57) **Abstract**

The invention relates to a protector for stomata made up of two rectangular sheets with a small, likewise rectangular void in their central area joined at one of the ends, being cover and base, respectively, which fixes to the lower face of the upper sheet or cover and at the same angle of attachment a third sheet, this sheet being longitudinally shorter, narrow and made from a transpirable material like a filter, with the end opposite to the fixed end being free like a tab, which fits into a strap arranged for such purpose on the inner face of the base sheet. Emerging from the upper face of the cover, above the central void, there is a final sheet which, in terms of size and shape, acts as a diffusing shield with the four sides being somewhat open.

## Description

### Object of the Invention

The present specification relates to an invention patent relating to a protector for stomata for its particular use in those people in whom a laryngectomy has been performed, who have undergone an operation for the surgical removal of the larynx, and the evident purpose of which is to provide a total quality service in a completely hygienic manner as to the patient's needs, being a single-use disposable sterilized element, as well as to provide the most hygienic handling possible, through a system that is simple in its implementation and assembly for its placement and simplified handling, as well as inexpensive in its production, which allows obtaining a much more hygienic, improved, safe and even aesthetic result for the patient's mood.

### Field of the Invention

This invention is applicable within the broad and extensive industry of the manufacture of sanitary and hospital accessories mainly used in hospitals, clinics, health centers, and mainly distributed in pharmacies, to cover the needs of people who have undergone a laryngectomy or similar surgical operations.

### Description of the Invention

The protector for stoma proposed by the invention is in and of itself an evident solution for the problems existing in this field today as it allows the necessary simple and completely hygienic handling of the protector, providing simple, fast and safe handling without losing any time, with a calibrated, accurate and safe result, starting from a simple and inexpensive product.

The current problems deriving from the need for the urgent use of protection systems for the stoma in people whose larynx has been surgically removed are very well known because with regard to the breathing function, these people go from breathing in air through their nose or mouth to perform this function through the stoma arranged at the base of the neck, this hole being proportional to the height of the person in question, and therefore the air introduced into their lungs is direct, i.e., unfiltered, unheated or unmoistened; therefore, since the operated area is considerably defenseless and has marked drawbacks, it requires a specific treatment and special care from the physical point of view. In psychological terms, the person involved has a different attitude in his or her social and family life, and also experiences a loss of self-esteem because he or she is aware of the impact, especially on a visual level, caused on other people and the feelings it produces.

The systems commonly used to reduce the foregoing to the greatest possible extent are usually uncomfortable and conspicuous since they denote and give away the person's situation and only worsen his or her condition both physically and psychologically. The most widely used system is based on placing a cloth or transpirable material like a bib, held behind the neck by means of bands tied to one another, in many cases being a clothing accessory or element. The main drawback is that since this element is continuously worn on the neck, it can cause a feeling of irritation and heat, in addition to its cost being easily identifiable and recognized by others. There are also adhesive filters which acceptably perform the filtering function, but like in the previous case, they are not very discrete and are expensive. It must further be added that given their adhesive condition, they complicate the cleaning operation when the user suffers a coughing episode or mucosal secretions.

The aforementioned problems are satisfactorily solved with the proposed system, which consists of a rectangular laminar cover with a central slot, where another sheet protecting the slot projects, having longitudinal openings on its four sides, acting as a diffusing shield. The cover in turn is bent at one of its ends with another transparent filament for clinical use, its lower face being adhesive in order to place it as a base on the neck, around the stoma; said base has a slot in its central area symmetrically with the cover, arranged between both and secured to the lower face of the cover by means of a strap at one end and secured at the other end there is a filter-like strip of transpirable material, therefore being practicable not only to be able to access and clean the stoma, but so that the filter can also be changed once it is rendered useless, in the exact same way and manner in which conventional adhesive dressings are used, the protector herein described is also entirely disposable. The composition and structural arrangement of each and every one of the elements makes any accidental aspiration thereof impossible.

The advantages which emerge from this type of protector for stomata are clear in view of the problems indicated above, and they mainly consist of the comfort and discretion in their placement, allowing the patient to live a much more natural life without having physical problems and greatly minimizing psychological problems because the number of others seeing the protector will be minimized, and at most they will see the positioning of an adhesive dressing that is virtually like those used to cover any injury.

### Description of the Drawings

To complement the description that is being made and for the purpose of aiding to better understand the features of the invention, a set of drawings is attached to this specification as an integral part thereof, in which the following is shown with an illustrative and nonlimiting nature:

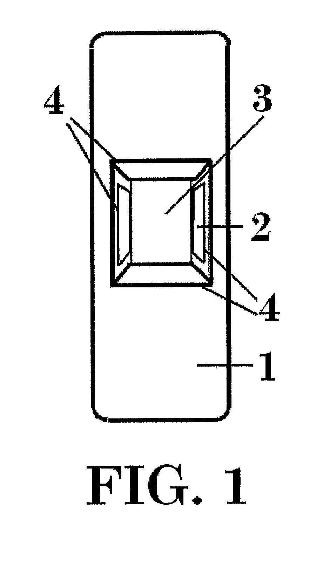

Figure 1 shows a plan view, as the object may be seen by others.

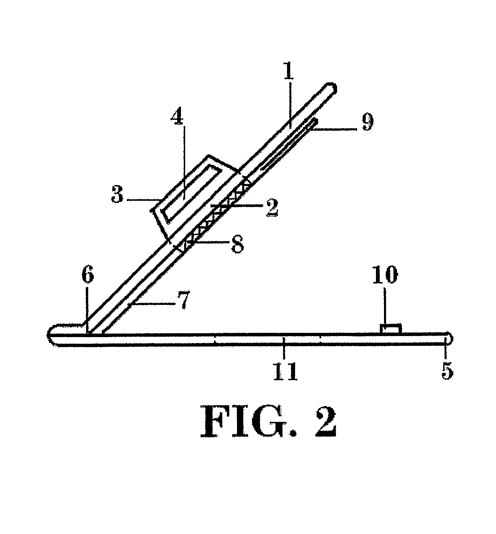

Figure 2 shows a profile view, the system being open.

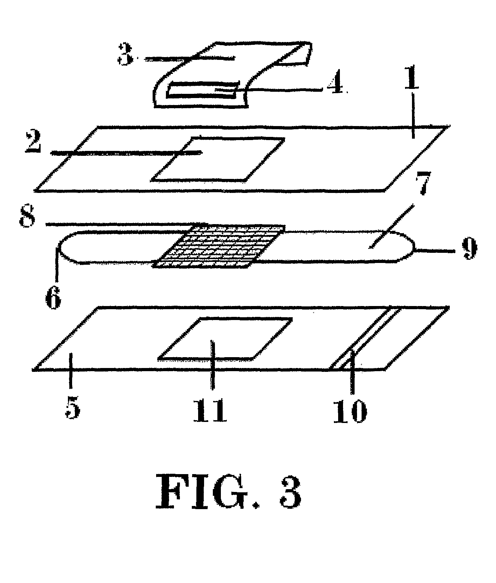

Figure 3 shows a perspective view of all the elements forming the invention separate from one another.

### Preferred Embodiment of the Invention

In view of the depicted figures, it can be seen that the invention relates to a rectangular laminar cover (1) with an also rectangular void (2) in the center where the diffusing shield (3) with opening (4) on its four sides is arranged. The lower face of the cover (1) is attached at one of its ends to the base sheet (5) and secured between them, the band or strip (7), formed in its central area by a transpirable material (8), is arranged at the end (6), the opposite end being free in the way of a tab (9), and once the cover (1) and the base (5) are closed and bent, said tab (9) will be fitted into the strap (10) arranged on the inner face of the base (5), which has, symmetrical to the cover (1), a void (11), the latter being that which is positioned around the stoma.

It is not considered necessary to make this description more extensive so that a person skilled in the art may understand the scope of the invention and the advantages derived therefrom.

The materials, shape, size and arrangement of the elements shall be susceptible to variation provided it does not alter the essential nature of the invention.

The terms used in this specification must always be interpreted broadly, not in a limiting manner.

## Claims

1. A protector for stomata, **characterized by** having a raised upper central part open at the four sides.

2. The protector for stomata according to claim 1, **characterized by** having a disposable strip or filter between the cover and the base.

3. The protector for stomata according to the previous claims, **characterized by** having on the inner face of the base a strap on one of its sides.

4. The protector for stomata according to the previous claims, **characterized by** having a rectangular void area in the center of the cover and of the base with a transpiration strip between them.
